(19) **Europäisches Patentamt**
**European Patent Office**
**Office européen des brevets**

(11) **EP 3 961 195 B1**

(12) **EUROPÄISCHE PATENTSCHRIFT**

(45) Veröffentlichungstag und Bekanntmachung des Hinweises auf die Patenterteilung:
**20.08.2025 Patentblatt 2025/34**

(21) Anmeldenummer: **20193394.2**

(22) Anmeldetag: **28.08.2020**

(51) Internationale Patentklassifikation (IPC):
*G01N 21/65* (2006.01)    *G01N 33/22* (2006.01)

(52) Gemeinsame Patentklassifikation (CPC):
**G01N 21/65; G01N 33/225**

(54) **MESSEINRICHTUNG ZUR BRENN- ODER HEIZWERTBESTIMMUNG EINES KOHLENWASSERSTOFFHALTIGEN BRENNGASES**

MEASURING DEVICE FOR DETERMINING THE CALORIFIC VALUE OF A HYDROCARBON-CONTAINING FUEL GAS

DISPOSITIF DE MESURE PERMETTANT DE DÉTERMINER LA VALEUR DE COMBUSTION OU DE CHAUFFAGE D'UN GAZ DE COMBUSTION CONTENANT DES HYDROCARBURES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**02.03.2022 Patentblatt 2022/09**

(73) Patentinhaber: **Siemens Aktiengesellschaft 80333 München (DE)**

(72) Erfinder:
• **Heffels, Camiel**
  **76297 Stutensee-Büchig (DE)**
• **Harr, Konstantin**
  **76131 Karlsruhe (DE)**

(74) Vertreter: **Siemens Patent Attorneys Postfach 22 16 34 80506 München (DE)**

(56) Entgegenhaltungen:
DE-A1- 19 921 981    US-A- 4 953 976

• CHRISTIAAN MUL: "Raman spectroscopy for natural gas process applications", 12 December 2017 (2017-12-12), XP055766938, Retrieved from the Internet <URL:https://esc.fnwi.uva.nl/thesis/centraal/files/f1221299931.pdf> [retrieved on 20210120]
• S C EICHMANN ET AL: "Determination of gas composition in a biogas plant using a Raman-based sensor system", MEASUREMENT SCIENCE AND TECHNOLOGY, IOP, BRISTOL, GB, vol. 25, no. 7, 16 June 2014 (2014-06-16), pages 75503, XP020266845, ISSN: 0957-0233, [retrieved on 20140616], DOI: 10.1088/0957-0233/25/7/075503
• FLORISSON O ET AL: "RAPID DETERMINATION OF THE WOBBE INDEX OF NATURAL GAS", JOURNAL OF PHYSICS E. SCIENTIFIC INSTRUMENTS, IOP PUBLISHING, BRISTOL, GB, vol. 22, no. 2, 1 February 1989 (1989-02-01), pages 123 - 128, XP000036441, ISSN: 0022-3735, DOI: 10.1088/0022-3735/22/2/009

EP 3 961 195 B1

**Beschreibung**

Die Erfindung betrifft ein Verfahren zur Brenn- oder Heizwertbestimmung eines kohlenwasserstoffhaltigen Brenngases.

Die Erfindung betrifft ferner eine Messeinrichtung zur Brennoder Heizwertbestimmung eines kohlenwasserstoffhaltigen Brenngases.

Die Norm DIN EN ISO 6976:2016 legt Verfahren für die Berechnung brenntechnisch relevanter Größen wie Brennwert, Heizwert, Dichte, relativer Dichte sowie oberem und unterem Wobbe-Index von Erdgasen, Erdgasaustauschgasen und sonstigen Brenngasen fest, wenn die Zusammensetzung der Gasgemische in Stoffmengenanteilen bekannt ist [1]. Anhand der festgelegten Verfahren können die Eigenschaften des Gasgemisches bei allgemein üblichen Referenzbedingungen berechnet werden.

Die Messung der Zusammensetzung von Erdgas erfolgt üblicherweise mit Hilfe eines Gaschromatographen (GC), in dem die Komponenten des Gasgemischs chromatographisch getrennt und nacheinander detektiert werden. Anhand der quantitativ bestimmten Komponenten können dann die interessierenden brenntechnisch relevanten Größen berechnet werden. Das ganze Verfahren dauert meist einige Minuten. Obwohl die Genauigkeit der Brennwertbestimmung mit einem Gaschromatographen bei < 0,1 % liegt, kann für Mischanwendungen und Gasturbinen eine schnellere Brennwertbestimmung erwünscht sein, die nicht ganz so präzise sein muss.

Die Zusammensetzung von Erdgas oder ähnlichen Gasen kann auch mittels Infrarot- oder Raman-Spektroskopie bestimmt werden [2], [3], [4]. Auch hier erfolgt die Berechnung interessierender brenntechnisch relevanter Größen aufgrund der zuvor quantitativ bestimmten Gaskomponenten.

DE 199 21 981 A1 [5] beschreibt ein Verfahren zum Betrieb einer Gasturbine. Dabei wird die der Gasturbine zugeführte Brenngasmenge basierend auf einem mittels Raman-Spektroskopie ermittelten Heizwert des Brenngases geregelt.

Die Raman-Spektroskopie basiert auf der inelastischen Streuung von Laserlicht an einer Probe. Die gestreute Strahlung (Raman-Strahlung) ist gegenüber dem einfallenden Licht in der Frequenz verschoben (Raman-Verschiebung) und wird beispielsweise mittels eines Spektrografen (siehe z.B. [5]) mit nachgeordnetem CCD-Array detektiert. Die Frequenzverschiebung zwischen dem einfallenden und dem gestreuten Licht ist charakteristisch für die Schwingungs- und Rotationsstruktur der chemischen Spezies der Probe. Die Raman-Spektren einer Spezies stellen einzigartige molekulare Fingerprints dar und können daher für analytische Zwecke verwendet werden.

In der Praxis können jedoch die spektrale Auflösung und Empfindlichkeit eines CCD-Arrays für die Ermittlung der Zusammensetzung des Brenngases nicht ausreichen, weil eine hohe spektrale Auflösung kleine Pixel erfordert, die dementsprechend nur schwache Signal erzeugen.

Der Erfindung liegt die Aufgabe zugrunde, eine schnelle Brenn- oder Heizwertbestimmung mit vergleichsweise geringem Aufwand zu ermöglichen.

Gemäß der Erfindung wird diese Aufgabe durch das in Anspruch 1 definierte Verfahren oder die in Anspruch 7 definierte Messeinrichtung gelöst, von denen vorteilhafte Weiterbildungen in den Unteransprüchen angegeben sind.

Gegenstand der Erfindung ist somit ein Verfahren zur Brennoder Heizwertbestimmung eines kohlenwasserstoffhaltigen Brenngases mittels eines Raman-Photometers, wobei die nach Interaktion von Laserlicht mit dem Brenngas erhaltene Raman-Strahlung mittels eines Bandpassfilters auf einen Wellenzahlbereich der C-H-Streckschwingungen der in dem Brenngas enthaltenen Kohlenwasserstoffe um 2900 cm$^{-1}$ begrenzt und einem Photomultiplier zugeführt und von diesem integrativ detektiert wird, und wobei der Brenn- oder Heizwert aus dem Ausgangssignal des Photomultipliers ermittelt wird;

wobei die Raman-Strahlung mittels eines weiteren Bandpassfilters auf einen Wellenzahlbereich der C-C-Schwingungen der in dem Brenngas enthaltenen Kohlenwasserstoffe mit zwei oder mehr Kohlenstoffatomen um 990 cm$^{-1}$ begrenzt und einem weiteren Photomultiplier zugeführt und von diesem integrativ detektiert wird, und wobei der aus dem Ausgangssignal des Photomultipliers ermittelte Brenn- oder Heizwert mit dem Ausgangssignal des weiteren Photomultipliers korrigiert wird.

Gegenstand der Erfindung ist ferner eine Messeinrichtung zur Brenn- oder Heizwertbestimmung eines kohlenwasserstoffhaltigen gemäß Anspruch 7.

Gemäß der Erfindung wird anstelle eines Raman-Spektrometers ein Raman-Photometer verwendet. Anders als der Ansatz, alle Hauptkomponenten in dem Brenngas mittels eines Raman-Spektrometers zu bestimmen, erfolgt die Bestimmung des Brenn- oder Heizwertes über ein spektroskopisches Integralverfahren. Die Erfindung beruht auf der Feststellung, dass das Flächenintegral der Raman-Signaturen (im Folgenden als Raman-Signal bezeichnet) der in dem Brenngas enthaltenen Kohlenwasserstoffe in dem engen Bereich der von den C-H-Streckschwingungen verursachte Raman-Verschiebung von 2900 cm$^{-1}$ mit dem Brennwert und dem (in der Praxis weniger relevanten) Heizwert des Brenngases korreliert.

Bei den von der erfindungsgemäßen Brenn- oder Heizwertbestimmung umfassten Kohlenwasserstoffen handelt es sich insbesondere um Methan und Ethen sowie die höheren Alkane und ihre Isomere bis Pentan.

Die Brenn- oder Heizwertbestimmung kann bei einem erhöhten Druck des Brenngases von 0.1 MPa (1 bar) absolut bis zu 1 MPa (10 bar) absolut, vorzugsweise bei mindestens 0.5 MPa (5 bar) absolut, erfolgen, um ein ausreichend starkes Raman-Signal zu erhalten.

Die Brenn- oder Heizwertbestimmung kann weiterhin vorzugsweise bei einer Wellenlänge des Laserlichts von 405 nm $\pm$ 10 nm erfolgen, wobei die Raman-Signaturen rund um 2900 cm$^{-1}$ mittels eines Bandpassfilters mit einer Zentralwellenlänge von 459 nm $\pm$ 13 nm und einer Halbwertsbreite von 5 bis 10 nm selektiv erfasst werden. Die kurze Laser-Wellenlänge von 405 nm verursacht in Gasen keine Fluoreszenz und führt zu einer guten Ausbeute von Raman-Photonen. Außerdem sind entsprechende Laser als Diodenlaser günstig und mit ausreichender optischer Leistung verfügbar. Die Streuung bei Lasern wird oft mit $\pm$ 5 nm angegeben.

Die Raman-Anregung kann alternativ bei einer Laser-Wellenlänge von 450 nm $\pm$ 10 nm stattfinden, wobei dann die Zentralwellenlänge des Bandpassfilters bei 518 nm liegt. Der Vorteil der längeren Wellenlänge wäre, dass das Bandpassfilter die Banden schärfer selektiert.

Um bei Brenngasen mit vergleichsweise hohem oder niedrigem Methananteil die lineare Regression der Korrelation zwischen dem Raman-Signal und dem Brenn- oder Heizwert zu verbessern, wird zusätzlich zu dem Flächenintegral der Raman-Signaturen der C-H-Streckschwingungen auch das Flächenintegral der Raman-Signaturen der Kohlenwasserstoffe mit mehr als einem Kohlenstoffatom (d. h. alle Kohlenwasserstoffe außer Methan) rund um die C-C-Rumpfschwingung mit einer breiten Raman-Verschiebung um 990 cm$^{-1}$ hinzugezogen.

Durch eine Erweiterung des Raman-Photometers mit einem zusätzlichen Messkanal zur Ermittlung der Konzentration von Wasserstoff anhand seiner Raman-Verschiebung von etwa 4155 cm$^{-1}$ kann auch der Brennwert von wasserstoffhaltigen Kohlenwasserstoffgemischen bestimmt werden. Damit können Anwendungen im Bereich der Wasserstoffbeimischung zum Erdgas oder Biomethan und Biogaseinspeisung in Erdgasnetze erschlossen werden.

Im Weiteren wird die Erfindung anhand von Ausführungsbeispielen und unter Bezugnahme auf die Figuren der Zeichnung erläutert; im Einzelnen zeigen

Fig. 1  ein Beispiel für die erfindungsgemäße Messeinrichtung mit einem Raman-Photometer,

Fig. 2  eine perspektivische Ansicht des Raman-Photometers,

Fig. 3  die Raman-Signatur von Kohlenwasserstoffen rund um eine Raman-Verschiebung von 2900 cm$^{-1}$ und

Fig. 4  ein Beispiel für die Korrelation der mit dem Raman-Photometer erhaltenen Raman-Signale mit den Brennwerten unterschiedlicher Erdgasgemische.

Gleiche Bezugszeichen haben in den verschiedenen Figuren die gleiche Bedeutung. Die Darstellungen sind rein schematisch und repräsentieren keine Größenverhältnisse.

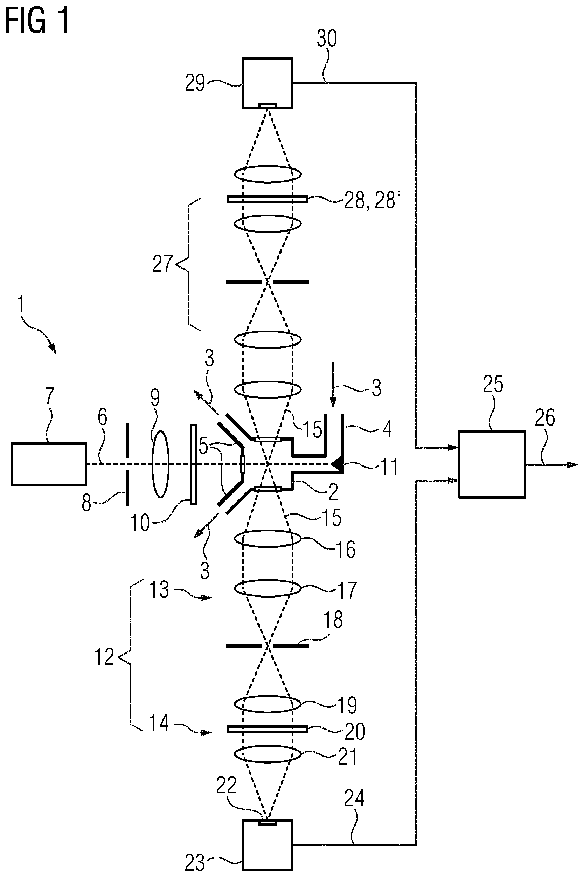

Fig. 1 zeigt in vereinfachter schematischer Darstellung eine Messeinrichtung mit einem Raman-Photometer 1.

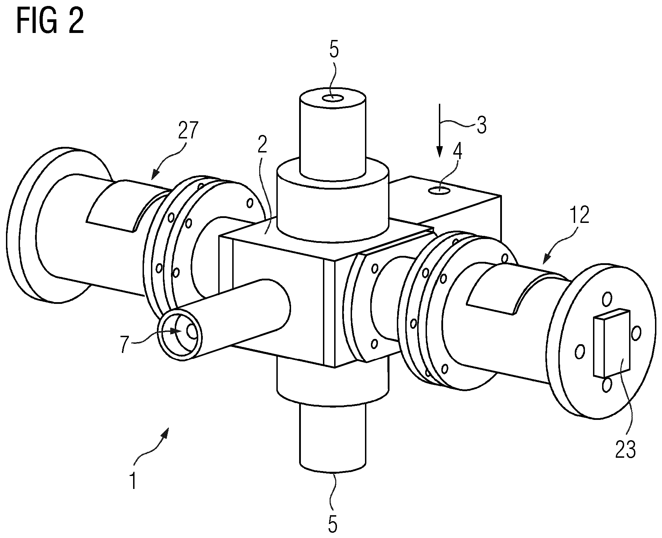

Fig. 2 zeigt das Raman-Photometer 1 in perspektivischer Darstellung.

Das Raman-Photometer 1 weist eine druckfeste (1 MPa (10 bar)) Messzelle 2 auf, die von einem Brenngas 3, z. B. Erdgas, mit erhöhtem geregeltem Druck bis zu 1 MPa (10 bar) absolut durchströmt wird. Das Brenngas 1 tritt über einen Gaseinlass 4 in die Messzelle 2 ein und verlässt diese an zwei Gasauslässen 5, wobei die Gasauslässe 5 entgegen der schematischen Darstellung in Fig. 1 vorzugsweise nach oben und unten aus der Zeichenebene herausführen (vgl. Fig. 2). Der Lichtstrahl 6 eines Lasers 7 fällt durch eine Blende 8, über eine Fokussierlinse 9 und ein Interferenzfilter 10 in die Messzelle 2 und durchstrahlt diese in Richtung zu dem Gaseinlass 4, wo er in einer rückstreuungsfreien Lichtfalle 11 aufgefangen wird. Der durch die Linse 9 fokussierte Linienabschnitt (Strahltaille) des Lichtstrahls (Gaußstrahl) 6 ist in der Mitte der Messzelle 2 positioniert.

Rechtwinklig zu dem Laserstrahl 6 ist an der Messzelle 2 eine erste Empfangsoptik 12 für die an den Molekülen des Brenngases 3 gestreuten Raman-Photonen angeordnet. Die Empfangsoptik 12 besteht aus zwei Stufen 13, 14, wobei in der ersten Stufe 13 das gestreute Licht 15 mithilfe von einer oder mehreren Linsen 16, 17 auf eine rechteckige Blende 18 von ca. 1 x 4 mm fokussiert wird. Hierdurch erfolgt eine räumliche Filterung des gestreuten Lichts 15, so dass in die zweite Stufe 14 nur solche Photonen gelangen, die aus einem begrenzten Volumen rund um den fokussierten Linienabschnitt des Laserstrahls 6 gestreut wurden. Die erste Linse 16 kann als druckfester Abschluss der Messzelle 2 dienen.

In der zweiten Stufe 14 durchläuft das mittels einer Linse 19 parallelisierte gestreute Licht 15 ein Bandpassfilter 20 in Form eines schmalbandigen Interferenzfilters oder ggf. zweier hintereinander liegender Interferenzfilter. Die von dem Bandpassfilter 20 gaskomponentenspezifisch selektierten Photonen werden mittels einer weiteren Linse 21 auf einer rechteckigen Photokathode 22 (ca. 1 x 4 mm) eines Photomultipliers 23 abgebildet, mittels dessen sie einzelnen nachgewiesen werden. Der Photomultiplier 23 erzeugt ein Ausgangssignal (Raman-Signal) 24, das zu der Anzahl der von der Photokathode 22 absorbierten Photonen pro Zeiteinheit proportional ist und einer Auswerteeinrichtung (Prozessor) 25 zur Auswertung und Ermittlung sowie Ausgabe des Brennwertes 26 des Brenngases 3 zugeführt wird.

Es wird bevorzugt ein Laser 7 mit einer Wellenlänge von 405 nm verwendet. Diese kurze Wellenlänge verursacht in Gasen keine Fluoreszenz und führt zu einer guten Ausbeute von Raman-Photonen. Außerdem ist der entsprechende Laser 7 als Diodenlaser günstig verfügbar. Die Laserleistung kann zur Erfüllung des Ex-Schutzes auf unter 35 mW begrenzt werden, wobei eine Nachweisgrenze für Methan < 100 ppm bei einer Messdauer von einer Sekunde erreicht werden kann. Die Strahlqualität und Linienbreite des Laserstrahls 6 wird durch die Blende 8 und das schmalbandige Interferenzfilter 10 verbessert, so dass das Hintergrundsignal in der Messzelle 2 auf ein niedriges Niveau gebracht wird. Das Hintergrundsignal wird zudem durch die Anordnung und Gestaltung der Gasauslässe 5 und der Lichtfalle 11 stark reduziert. Der Photomultiplier 23 dient dazu, die, insbesondere auch wegen der geringen Laserleistung, in nur sehr geringer Anzahl erzeugten Raman-Photonen in ein ausreichend starkes Raman-Signal 24 umzuwandeln. Außerdem erfolgen die Messungen bei einem erhöhten Druck in der Messzelle 2 von, z. B., 0.5 MPa (5 bar) absolut, weil die Anzahl der erzeugten Raman-Photonen und damit das Raman-Signal 24 proportional mit dem Messdruck ansteigen. Dazu kann an den, ggf. zusammengeführten, Gasauslässen 5 ein Druckregler vorgesehen sein.

Um die Messeinrichtung in einer industriellen Umgebung mit höherer Laserleistung (> 35 mW) betreiben zu können, kann die Messzelle 2 auch in druckfester Kapselung (Ex-d) ausgeführt sein.

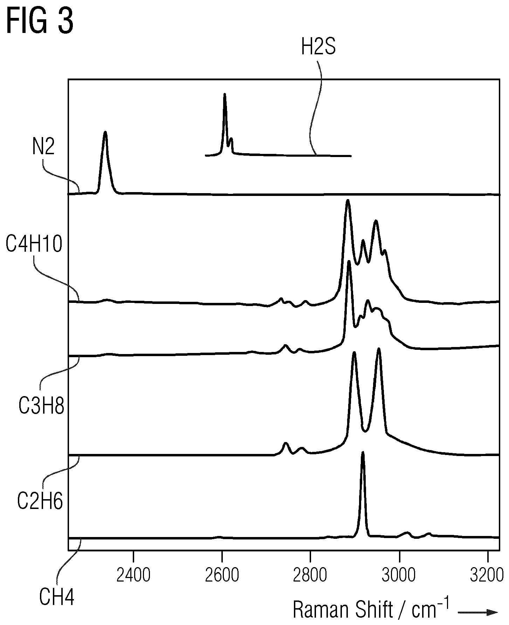

Fig. 3 zeigt einen Ausschnitt aus den Raman-Spektren der Hauptbestandteile von Erdgas, hier Methan, Ethan, Propan und Butan, und den Nebenbestandteilen Stickstoff und Schwefelwasserstoff. Im Erdgas sind auch noch höhere Kohlenwasserstoffe und diverse Isomere in geringe Konzentration < 0,1 % enthalten. Wie gezeigt, liegen die von den C-H-Streckschwingungen der Kohlenwasserstoffe erzeugten Raman-Verschiebungen bei etwa 2900 cm$^{-1}$. Sie sind für alle Kohlenwasserstoffe etwa gleich und frei von jeglichen anderen chemischen Bindungen in dem Erdgasgemisch 3. Für den Zusammenhang zwischen der Zentralwellenlänge $\lambda$ des Bandpassfilters 20, der Laserwellenlänge $\lambda_{ex}$ und der Raman-Verschiebung (Raman Shift) RS gilt:

$$1/\lambda[\mathrm{nm}] = 1/\lambda_{ex}[\mathrm{nm}] - RS[\mathrm{cm}^{-1}]/10^7 \; .$$

.

Bei dem hier beschriebenen Ausführungsbeispiel des Raman-Photometers 1 ist das Bandpassfilter 20 mit einer Zentralwellenlänge von etwa 459 nm und einer Halbwertsbreite (Full Width at Half Maximum, FWHM) von 5 bis 10 nm so gewählt, dass die erwähnten C-H-Streckschwingungen erfasst werden.

Die Bestimmung des Brennwerts mit dem Raman-Photometer 1 erfolgt also nicht über die Bestimmung der einzelnen brennbaren Komponenten des Brenngases, sondern über eine integrale Messung der Konzentration der "brennbaren chemischen Bindungen" in den betreffenden Kohlenwasserstoffen. Hauptsächlich sind dies die C-H-Bindungen, die mit dem Bandpassfilter 20 anhand ihrer Raman-Verschiebungen um 2900 cm-1 in der Summe erfasst werden. Es wurde festgestellt, dass das Raman-Signal 24 mit dem Brennwert des Erdgases 3 korreliert. Die Korrelation kommt dadurch zustande, dass Ethan, Propan und höhere Kohlenwasserstoffe aufgrund der erhöhten Zahl von C-H-Bindungen pro Volumeneinheit einen höheren Raman-Wirkungsquerschnitt aufweisen und daher ein höheres Streulichtsignal am Flächenintegral der Raman-Signatur rund um die Raman-Verschiebung von 2900 cm$^{-1}$ erzeugen. Für eine optimale Korrelation mit dem Brennwert müssen daher wie oben bereits erwähnt die Lage der Zentralwellenlänge und die Halbwertsbreite des Bandpassfilters 20 richtig gewählt werden, so dass insbesondere bei den höheren Alkanen die gesamte Signatur erfasst wird.

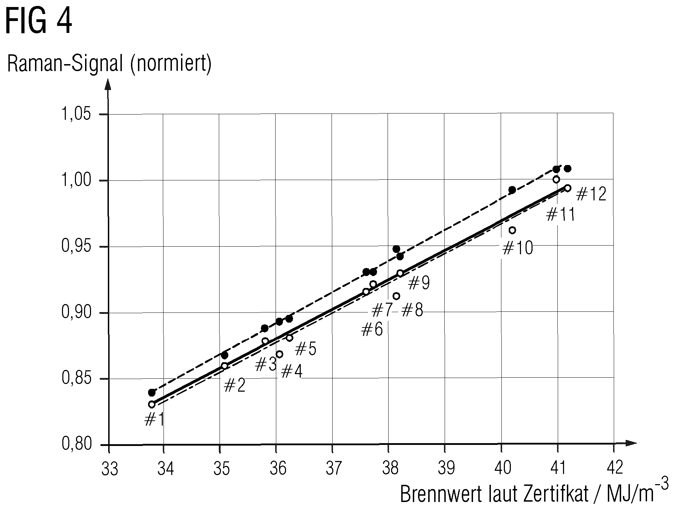

In Fig. 4 ist die Korrelation anhand von für 12 Erdgasmischungen mit bekannten Brennwerten erhaltenen Raman-Signalen 24 graphisch dargestellt. Die Messungen #1 bis #12 erfolgten bei einem Druck von 5000 hPa absolut, einem Durchfluss des Messgases 3 durch die Messkammer 2 von 1000 ml/min und einer auf konstant 25 °C geregelten Temperatur. Die Brennwertangaben wurden von dem Lieferanten der Erdgasmischungen mittels einer GC-Analyse bestimmt und auf den Analysenzertifikaten angegeben. Das Hintergrundsignal von Stickstoff (nur etwa 0,01% des C-H-Raman-Signals) wurde von den Raman-Signalen 24 subtrahiert. Die Regressionsgerade der 12 Messpunkte (mit weißer Füllung) ist durchgezogen gezeichnet. Unter den 12 Messpunkten gibt es vier deutliche Ausreißer, von denen die unter der Regressionsgeraden liegenden Ausreißer #4, #8, #10 von den Erdgasmischungen mit den geringsten Methananteilen resultieren und der Ausreißer #11 über der Regressionsgeraden von der Erdgasmischung mit dem höchsten Methananteil resultiert. Aus diesen Ergebnissen folgt, dass die höheren Alkane im Raman-Messverfahren etwas unterbewertet bleiben. Eine Aufweitung des Bandpassfilters 20 könnte eine Verbesserung des Korrelationskoeffizienten bringen. Aus der Messung von Fig. 4 kann eine Unsicherheit in der Bestimmung des Brennwerts mit Hilfe des Raman-Messverfahrens von 1,5 % abgeleitet werden. Lässt man die Ausreißer #4, #8, #10, #11 außer Acht, so erhält man die strichpunktierte Regressionsgerade. Die Unsicherheit der Brennwertbestimmung beträgt dann gemäß der größten Abweichung nur noch 0,5% vom Brennwert laut Zertifikat.

Anstelle der Nichtberücksichtigung der Ausreißer #4, #8, #10, #11 können deren Werte auch in Abhängigkeit von der MethanKonzentration in dem Brenngas 3 korrigiert werden. Dazu kann die Methan-Konzentration beispielsweise mit Hilfe eines selektiven nichtdispersiven Infrarot (NDIR-) Gasanalysators separat gemessenen werden.

Erfindungsgemäß, werden die Messwerte #1 bis #12 durch eine Höhergewichtung aller Kohlenwasserstoffe außer Methan korrigiert, indem die Raman-Strahlung 15 in einer zweiten Empfangsoptik 27 mittels eines weiteren Bandpassfilters (Interferenzfilter) 28 auf einen Wellenzahlbereich der C-C-Schwingungen der in dem Brenngas 3 enthaltenen Kohlenwasserstoffe mit mehr als einem Kohlenstoffatom um 990 cm$^{-1}$ begrenzt und in einem zweiten Photomultiplier 29 integrativ detektiert wird. Die zweite Empfangsoptik 27 ist an der Messzelle 2 gegenüber der ersten Empfangsoptik 12 angebaut und mit dieser außer dem Bandpassfilter 28 baugleich. Die Korrektur kann in einfacher Weise dadurch erfolgen, dass in der Auswerteeinrichtung 25 das Ausgangssignal (Raman-Signal) 30 des zweiten Photomultipliers 29 dem Ausgangssignal 24 des Photomultipliers 23 mit einem Kalibrierfaktor hinzuaddiert wird. Die korrigierten Messpunkte sind in Fig. 4 mit schwarzer Füllung und ihre Regressionsgerade gestrichelt dargestellt.

Sollte künftig eine Beimischung von Wasserstoff zum Erdgas Anwendung finden (z. B. Speicherung regenerativer Energie im Erdgasnetz), muss bei der Brennwertbestimmung zusätzlich auch die Konzentration des Wasserstoffs in dem Gasgemisch 3 berücksichtigt werden. Die Raman-Verschiebung bei 4155 cm$^{-1}$ von Wasserstoff ist dafür sehr gut geeignet. Durch einfaches Austauschen des Bandpassfilters 28 in der zweiten Empfangsoptik 27 gegen ein anderes geeignetes Bandpassfilter 28' oder mit Hilfe eines die jeweils benötigten Bandpassfilter enthaltenden Filterschiebers oder Filterrads können daher neben Wasserstoff auch andere Komponenten wie Kohlendioxid, Stickstoff und Schwefelwasserstoff gemessen werden. So können durch Einbeziehung einer Stickstoffmessung oder einer Kohlendioxidmessung ggf. genauere Ergebnisse der Brenn- oder Heizwertwertbestimmung über einen größeren Bereich der Methankonzentration erreicht werden. Die benötigte Zentralwellenlängen der entsprechenden Bandpassfilter sind in der folgenden Tabelle aufgelistet.

| Gaskomponente | Raman-Verschiebung [cm$^{-1}$] | relativer Raman-Streuquerschnitt | Zentralwellenlänge der Raman-Bande [nm] bei Anregung mit | |
|---|---|---|---|---|
| | | | 405 nm | 450 nm |
| H2 (Wasserstoff) | 4155 | 3, 9 | 487 | 554 |
| N2 (Stickstoff) | 2331 | 1,0 | 447 | 503 |
| CO2 (Kohlendioxid) | 1388 | 1, 1 | 429 | 480 |
| CH4 (Methan) | 2917 | 8, 6 | 459 | 518 |
| C2H6 (Ethan) | 2914 | 15,0 | 459 | 518 |
| C2H4 (Ethen) | 3020 | 6,4 | 461 | 521 |
| C3H8 (Propan) | 2908 | 19,6 | 459 | 518 |
| C4H10 (n-Butan) | 2890 | 15.6 | 459 | 517 |
| C4H10 (Isobutan) | 2880 | 8,4 | 458 | 517 |
| H2S (Schwefelwasserstoff) | 2610 | | 453 | 510 |

Zur gleichzeitigen Messung der unterschiedlichen Komponenten kann das Raman-Photometer 1 auch mit mehr als den hier gezeigten zwei Empfangsoptiken 12, 27 ausgerüstet sein, wobei die weiteren Empfangsoptiken insbesondere in Richtung senkrecht zur Zeichenebene an der Messzelle 2 angebaut sein können.

Lediglich aus Gründen der einfacheren Darstellung sind in Fig. 1 die Fenster, durch die der Laserstrahl 6 in die Messzelle 2 eintritt und die Raman-Strahlung 15 aus der Messzelle 2 austritt, nicht gezeigt. Die Fenster können durch das Interferenzfilter 10 und Linsen, z. B. 16, der Empfangsoptiken 12, 27 gebildet werden.

Das erfindungsgemäße Raman-Messverfahren kann mit einer Reihe von Kalibriergasen einfach kalibriert werden. Die Messung wird am kontinuierlichen Gasstrom durchgeführt, wobei das Ergebnis im Sekundentakt zur Verfügung steht.

Referenzen:

[1] Norm DIN EN ISO 6976:2016: Erdgas - Berechnung von Brenn- und Heizwert, Dichte, relativer Dichte und Wobbeindex aus der Zusammensetzung

[2] EP 1 174 705 A1

[3] Johannes Kiefer: Recent Advances in the Characterization of Gaseous and Liquid Fuels by Vibrational Spectroscopy. Energies 2015, 8, 3165-3197

[4] Christiaan Mul, Master Thesis: Raman spectroscopy for natural gas process applications - An instrumental and operational survey of theory and practice, 12.12.2017, Vrije Universiteit Amsterdam, Analytical Solutions and Products B.V.

[5] DE 199 21 981 A1

**Patentansprüche**

1.  Verfahren zur Brenn- oder Heizwertbestimmung eines kohlenwasserstoffhaltigen Brenngases (3) mittels eines Raman-Photometers (1), wobei die nach Interaktion von Laserlicht (6) mit dem Brenngas (3) erhaltene Raman-Strahlung (15) mittels eines Bandpassfilters (20) auf einen Wellenzahlbereich der C-H-Streckschwingungen der in dem Brenngas enthaltenen Kohlenwasserstoffe um 2900 cm$^{-1}$ begrenzt und

    einem Photomultiplier (23) zugeführt und von diesem integrativ detektiert wird, und wobei der Brenn- oder Heizwert (26) aus dem Ausgangssignal (24) des Photomultipliers (23) ermittelt wird; wobei die Raman-Strahlung (15) mittels eines weiteren Bandpassfilters (28) auf einen Wellenzahlbereich der C-C-Schwingungen der in dem Brenngas (3) enthaltenen Kohlenwasserstoffe mit zwei oder mehr Kohlenstoffatomen um 990 cm$^{-1}$ begrenzt und einem weiteren Photomultiplier (29) zugeführt und von diesem integrativ detektiert wird, und wobei der aus dem Ausgangssignal (24) des Photomultipliers (23) ermittelte Brenn- oder Heizwert (26) mit dem Ausgangssignal (30) des weiteren Photomultipliers (29) korrigiert wird.

2.  Verfahren nach Anspruch 1, **dadurch gekennzeichnet, dass** die Kohlenwasserstoffe die C1- bis C5-Alkane und ihre Isomere umfassen.

3.  Verfahren nach Anspruch 1 oder 2, **dadurch gekennzeichnet, dass** die Brenn- oder Heizwertbestimmung bei einem erhöhten Druck des Brenngases (3) von 0.1 MPa absolut bis zu 1 MPa absolut, vorzugsweise bei mindestens 0.5 MPa erfolgt.

4.  Verfahren nach einem der vorangehenden Ansprüche, **dadurch gekennzeichnet, dass** die Brenn- oder Heizwertbestimmung bei bei einer Wellenlänge des Laserlichts (6) von 405 nm $\pm$ 10 nm oder 450 nm $\pm$ 10 nm erfolgt.

5.  Verfahren nach Anspruch 4, **dadurch gekennzeichnet, dass** ein Bandpassfilter (20) mit einer Zentralwellenlänge von 459 nm $\pm$ 13 nm bzw. 518 nm $\pm$ 13 nm und einer Halbwertsbreite von 5 bis 10 nm verwendet wird.

6.  Verfahren nach einem der vorangehenden Ansprüche, **dadurch gekennzeichnet, dass** zur Brenn- oder Heizwertbestimmung eines neben Kohlenwasserstoffen auch Wasserstoff enthaltenden Brenngases (3) die Raman-Strahlung (15) mittels eines zusätzlichen Bandpassfilters (28') auf einen Wellenzahlbereich der um 4155 cm$^{-1}$ begrenzt und dem weiteren Photomultiplier (29) oder einem zusätzlichen Photomultiplier zugeführt und von diesem integrativ detektiert wird, und wobei der Brenn- oder Heizwert (26) aus dem Ausgangssignal (24) des Photomultipliers (23) und dem Ausgangssignal (30) des weiteren Photomultipliers oder zusätzlichen Photomultipliers (29) ermittelt wird.

7.  Messeinrichtung zur Brenn- oder Heizwertbestimmung eines kohlenwasserstoffhaltigen Brenngases (3),

    mit einem ein Laser (7), ein Bandpassfilter (20), eine von dem Brenngas (3) durchströmbare Messzelle (2) und einen Photomultiplier (23) enthaltenden Raman-Photometer (1), das dazu ausgebildet ist, die nach Interaktion von Laserlicht (6) mit dem Brenngas (3) erhaltene Raman-Strahlung (15) mittels des Bandpassfilters (20) auf einen Wellenzahlbereich der C-H-Streckschwingungen der in dem Brenngas (3) enthaltenen Kohlenwasserstoffe um 2900 cm$^{-1}$ zu begrenzen und dem Photomultiplier (23) zuzuführen, wobei das Raman-Photometer (1) ferner einen weiteren Bandpassfilter (28) und einen weiteren Photomultiplier (29) umfasst und dazu ausgebildet ist, die Raman-Strahlung mittels des weiteren Bandpassfilters (28) auf einen Wellenzahlbereich der C-C-Schwingungen der in dem Brenngas enthaltenen Kohlenwasserstoffe mit zwei oder mehr Kohlenstoffatomen um 990 cm$^{-1}$ zu begrenzen und dem weiteren Photomultiplier (29) zuzuführen; wobei die Messeinrichtung eine Auswerteeinrichtung (25) umfasst, die dazu eingerichtet ist, den Brenn- oder Heizwert (26) aus dem Ausgangssignal (24) des Photomultipliers (23) zu ermitteln und mit dem Ausgangssignal (30) des weiteren Photomultipliers (29) zu korrigieren.

**Claims**

1. Method for determining the gross or net calorific value of a hydrocarbon-containing fuel gas (3) by means of a Raman photometer (1), wherein the Raman radiation (15) obtained following interaction of laser light (6) with the fuel gas (3) is limited by means of a bandpass filter (20) to a wavenumber range of the C-H stretching vibrations of the hydrocarbons contained in the fuel gas around 2900 cm$^{-1}$ and is supplied to a photomultiplier (23) and integratively detected by the latter, and wherein the gross or net calorific value (26) is determined from the output signal (24) of the photomultiplier (23) ;
wherein the Raman radiation (15) is limited by means of a further bandpass filter (28) to a wavenumber range of the C-C vibrations of the hydrocarbons contained in the fuel gas (3) and having two or more carbon atoms around 990 cm$^{-1}$ and is supplied to a further photomultiplier (29) and integratively detected by the latter, and wherein the gross or net calorific value (26) determined from the output signal (24) of the photomultiplier (23) is corrected by means of the output signal (30) of the further photomultiplier (29).

2. Method according to claim 1, **characterised in that** the hydrocarbons comprise the C1 to C5 alkanes and their isomers.

3. Method according to claim 1 or 2, **characterised in that** the gross or net calorific value is determined at an increased pressure of the fuel gas (3) of 0.1 MPa absolute up to 1 MPa absolute, preferably at no less than 0.5 MPa.

4. Method according to one of the preceding claims, **characterised in that** the gross or net calorific value is determined at a wavelength of the laser light (6) of 405 nm $\pm$ 10 nm or 450 nm $\pm$ 10 nm.

5. Method according to claim 4, **characterised in that** a bandpass filter (20) having a central wavelength of 459 nm $\pm$ 13 nm or 518 nm $\pm$ 13 nm and a full width at half maximum of 5 to 10 nm is used.

6. Method according to one of the preceding claims, **characterised in that** in order to determine the gross or net calorific value of a fuel gas (3) also containing hydrogen in addition to hydrocarbons, the Raman radiation (15) is limited by means of an additional bandpass filter (28') to a wavenumber range around 4155 cm$^{-1}$ and is supplied to the further photomultiplier (29) or an additional photomultiplier and integratively detected by the latter, and wherein the gross or net calorific value (26) is determined from the output signal (24) of the photomultiplier (23) and the output signal (30) of the further photomultiplier or additional photomultiplier (29).

7. Measuring instrument for determining the gross or net calorific value of a hydrocarbon-containing fuel gas (3), comprising a Raman photometer (1) containing a laser (7), a bandpass filter (20), a measuring cell (2) through which the fuel gas (3) can pass and a photomultiplier (23), which Raman photometer (1) is embodied to limit the Raman radiation (15) obtained following interaction of laser light (6) with the fuel gas (3) by means of the bandpass filter (20) to a wavenumber range of the C-H stretching vibrations of the hydrocarbons contained in the fuel gas (3) around 2900 cm$^{-1}$ and to supply the same to the photomultiplier (23), wherein

   the Raman photometer (1) further comprises a further bandpass filter (28) and a further photomultiplier (29) and is embodied to limit the Raman radiation by means of the further bandpass filter (28) to a wavenumber range of the C-C vibrations of the hydrocarbons contained in the fuel gas having two or more carbon atoms around 990 cm$^{-1}$ and to supply this to the further photomultiplier (29); wherein
   the measuring instrument comprises an evaluation device which is configured to determine the gross or net calorific value (26) from the output signal (24) of the photomultiplier (23) and to correct it by means of the output signal (30) of the further photomultiplier (29).

**Revendications**

1. Procédé de détermination de la valeur de combustion ou de chauffage d'un gaz (3) combustible contenant des hydrocarbures au moyen d'un photomètre (1) Raman, dans lequel on limite le rayonnement (15) Raman, obtenu après interaction de lumière (6) laser avec le gaz (3) combustible, au moyen d'un filtre (20) passe-bande à un domaine de nombre d'onde des vibrations de valence C-H des hydrocarbures contenus dans le gaz combustible autour de 2900 cm$^{-1}$, et on l'envoie à un photomultiplicateur (23) et on le détecte par celui-ci par intégration, et dans lequel on détermine la valeur (26) de combustion ou de chauffage à partir du signal (24) de sortie du photomultiplicateur (23) ; dans lequel on limite le rayonnement (15) Raman, au moyen d'un autre filtre (28) passe-bande, à un domaine de nombre d'onde des vibrations C-C des hydrocarbures contenus dans le gaz (3) combustible ayant deux ou plusieurs atomes de carbone autour de 990 cm$^{-1}$, et on l'envoie à un autre photomultiplicateur (29) et on le détecte par celui-ci par intégration, et dans lequel on corrige la valeur (26) de combustion ou de chauffage, déterminée à partir du signal (24) de sortie du photomultiplicateur (23), par le signal (30) de sortie de l'autre photomultiplicateur (29).

2. Procédé suivant la revendication 1, **caractérisé en ce que** les hydrocarbures comprennent les alcanes en C1 à C5 et leurs isomères.

3. Procédé suivant la revendication 1 ou 2, **caractérisé en ce que** la détermination de la valeur de combustion ou de chauffage s'effectue à une pression élevée du gaz (3) combustible de 0,1 MPa absolu jusqu'à 1 MPa absolu, de préférence à au moins 0,5 MPa.

4. Procédé suivant l'une des revendications précédentes, **caractérisé en ce que** la détermination de la valeur de combustion de chauffage s'effectue à une longueur d'onde de la lumière (6) laser de 405 nm $\pm$ 10 nm ou de 450 nm $\pm$ 10 nm.

5. Procédé suivant la revendication 4, **caractérisé en ce que** l'on utilise un filtre (20) passe-bande ayant une longueur d'onde centrale de 459 nm $\pm$ 13 nm ou de 518 nm $\pm$ 13 nm et une largeur à mi-hauteur de 5 à 10 nm.

6. Procédé suivant l'une des revendications précédentes, **caractérisé en ce que**, pour la détermination de la valeur de combustion ou de chauffage d'un gaz (3) combustible contenant outre des hydrocarbures également de l'hydrogène, on limite le rayonnement (15) Raman, au moyen d'un filtre (28') passe-bande supplémentaire, à un domaine de nombre d'onde autour de 4155 cm$^{-1}$, et on l'envoie à l'autre photomultiplicateur (29) ou à un photomultiplicateur supplémentaire et on le détecte par celui-ci par intégration, et dans lequel on détermine la valeur (26) de combustion ou de chauffage à partir du signal (24) de sortie du photomultiplicateur (23) et du signal (30) de sortie de l'autre photomultiplicateur ou du photomultiplicateur (29) supplémentaire.

7. Dispositif de mesure pour la détermination de la valeur de combustion ou de la valeur de chauffage d'un gaz (3) combustible contenant un hydrocarbure, comprenant un photomètre (1) Raman comportant un laser (7), un filtre (20) passe-bande, une cellule (2) de mesure, dans laquelle peut passer le gaz (3) combustible, et un photomètre (1) Raman contenant un photomultiplicateur (23), le photomètre étant constitué pour limiter le rayonnement (15) Raman, obtenu après interaction de la lumière (6) laser avec le gaz (3) combustible, au moyen du filtre (20) passe-bande à un domaine de nombre d'onde des vibrations de valence C-H des hydrocarbures contenus dans le gaz (3) combustible autour de 2900 cm$^{-1}$, et pour l'envoyer au photomultiplicateur (23), dans lequel le photomètre (1) Raman comprend en outre un autre filtre (28) passe-bande et un autre photomultiplicateur (29) et est constitué pour limiter le rayonnement Raman au moyen de l'autre filtre (28) passe-bande à un domaine de nombre d'onde des vibrations C-C des hydrocarbures contenus dans le gaz combustible ayant deux ou plusieurs atomes de carbone autour de 990 cm$^{-1}$, et à l'envoyer à l'autre photomultiplicateur (29) ; dans lequel le dispositif de mesure comprend un dispositif (25) d'évaluation, qui est agencé pour déterminer la valeur (26) de combustion ou de chauffage à partir du signal (24) de sortie du photomultiplicateur (23) et pour la corriger par le signal (30) de sortie de l'autre photomultiplicateur (29).

FIG 1

FIG 2

## FIG 3

FIG 4

Raman-Signal (normiert)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19921981 A1

- EP 1174705 A1

**In der Beschreibung aufgeführte Nicht-Patentliteratur**

- **JOHANNES KIEFER**. Recent Advances in the Characterization of Gaseous and Liquid Fuels by Vibrational Spectroscopy. *Energies*, 2015, vol. 8, 3165-3197

- Master Thesis: Raman spectroscopy for natural gas process applications - An instrumental and operational survey of theory and practice. **CHRISTIAAN MUL**. Analytical Solutions and Products B.V. Vrije Universiteit Amsterdam, 12 December 2017